# EUROPEAN PATENT SPECIFICATION

(11) **EP 1 572 541 B1**
(45) Date of publication and mention of the grant of the patent: **23.01.2008**
(21) Application number: 03774421.6
(22) Date of filing: 19.11.2003
(51) Int. Cl.: B65B 55/10, A61L 2/20

(54) **DEVICE AND METHOD FOR STERILIZING PACKAGES**
VORRICHTUNG UND VERFAHREN ZUR STERILISIERUNG VON VERPACKUNGEN
DISPOSITIF ET PROCEDE PERMETTANT DE STERILISER DES PAQUETS

(30) Priority: 13.12.2002 SE 0203692
(43) Date of publication of application: 14.09.2005
(73) Proprietor: Tetra Laval Holdings & Finance SA, 1009 Pully (CH)
(72) Inventor: Möller, Håkan, 223 61 Lund (SE); Mott, Laurence, 231 92 Trelleborg (SE); Mårtensson, Lars, 240 14 Veberöd (SE)
(74) Representative: Forsberg, Lars-Ake
(86) International application number: PCT/SE2003/001792
(87) International publication number: WO 2004/054883

(56) References cited:
- US-A- 3 783 581
- US-A- 3 899 862
- US-A- 5 258 162
- US-B1- 6 189 292

## Description

### Technical Field of the Invention

The present invention relates to a device for sterilization in production of packages, which is adapted for sterilization with a gaseous sterilizing agent kept in the gaseous phase throughout the sterilization process, said device comprising a heating zone, a sterilization zone and a venting zone. The present invention also relates to a method of sterilizing packages in production of the packages, said packages having an open end and a closed end, wherein a gaseous sterilizing agent is used and kept in the gaseous phase throughout the sterilization process.

### Background Art

Devices and methods of the above-mentioned kind are known in the art, e.g. from US-5 258 162. Here, packages are passed through a housing divided into zones. In the first zone, hot air is introduced for heating the packages. In the second zone, gaseous hydrogen-peroxide is introduced for sterilizing the heated packages. In the third and final zone, the packages are filled with sterile contents. The three zones are all contained in one housing and are not physically separated from each other. Therefore, it may be difficult to control the flow between the different zones.

EP-A-394 734 discloses another device and method for sterilizing objects. In the device and method described, a vertically displaceable hood is placed over the object to be sterilized. A negative pressure is produced in the hood and a sterilization gas is introduced. The fact that a displaceable hood is needed makes this device somewhat complex.

US-3 899 862 describes a process for continuously sterilizing, aseptically filling and closing containers in a low-moisture environment maintained at substantially ambient conditions comprising the steps of subjecting the containers to a first flushing treatment wherein dry gas moves relatively to said containers to flush away ambient air in and around said containers, subsequently subjecting the containers to a sterilizing treatment wherein said containers are sterilized by the action of hydrogen chloride gas, and subjecting the containers to a second flushing treatment wherein conditioned air moves relatively to said containers to remove free hydrogen chloride from the surface thereof. The document further comprises a process for continuously sterilising containers in a low moisture environment comprising the steps of placing the containers on a moving conveyor; moving the containers into a sterile tunnel within which a gaseous environment is maintained at slight over pressure; moving the containers past a sterilising agent dosing device; dosing the containers with a sterilising agent which does not substantially increase the temperature of the container; retaining the sterilising agent in the container for a time sufficient for sterilisation to take place, and possibly venting out residues of the sterilising agent from the packages before filling.

### Summary of the Invention

An object of the present invention is to provide a device for sterilizing packages essentially of the type described above, but which enables an even safer sterilization process.

A specific object of the invention is to provide a device which allows improved control of the gas flow in and between the different zones.

Another object is to provide a device which allows improved control of the heating of packages.

It is also an object of the present invention to provide a method of sterilizing packages, which is improved as compared to prior art methods.

Yet another object is to provide a method which allows better control of the flow of air and gas in and between the different zones where the sterilization takes place.

According to the invention, these objects are achieved by a device of the kind mentioned by way of introduction, which has been given the characteristics of claim 1. Preferred embodiments of the inventive device are defined in dependent claims 2-19.

The above-mentioned objects are also achieved by a method as defined in claim 20, preferred variants thereof being defined in claims 21-34.

The inventive device for sterilization in production of packages comprises means for maintaining a higher pressure in the sterilization zone than in the heating zone and venting zone. Thus, it is possible to ensure that any leakage of air and gas is from the sterilization zone towards the surrounding zones and not the other way round.

In one embodiment, the heating, sterilization and venting zones are separated from each other by means of partitionings having openings for the passage of packages. This makes it easier to control the conditions in each zone.

The device is preferably adapted for sterilization with a gaseous sterilizing agent in the form of gaseous hydrogen peroxide. Hydrogen peroxide is a useful and reliable sterilizing agent.

The device is preferably adapted to sterilize packages before filling of the packages, said packages having an open end and a closed end. It is convenient to sterilize packages before filling them, since this makes it possible to maintain the sterility of sterile contents.

The heating zone may comprise means for heating the packages to a temperature above a dew point of the sterilizing agent used in the sterilization zone. In this way, it may be ensured that the sterilizing agent does not condensate on the packages.

In one embodiment, the venting zone comprises means for venting away the sterilizing agent used in the sterilization zone from the packages after sterilization. This makes it possible to ensure that the packages are free from sterilizing agent before filling.

The device may further comprise means for controlling a flow of gaseous sterilizing agent in the sterilization zone, such that the gaseous sterilizing agent flows essentially in a direction from the open end of the packages towards the closed end of the packages. This reduces the risk of recontamination of the packages.

The means for controlling the flow of gaseous sterilizing agent are preferably arranged to introduce the gaseous sterilizing agent in a top portion of the sterilization zone and to evacuate the gaseous sterilizing agent in a bottom portion of the sterilization zone, maintaining a flow of gaseous sterilizing agent essentially from top to bottom. This provides a secure way of maintaining a flow from the open end of the packages towards the closed end of packages standing on their closed end.

The device may further comprise means for controlling a venting air flow in the venting zone, such that the venting air flows essentially in a direction from the open end of the packages towards the closed end of the packages. Thus, the risk of recontamination of the sterilized packages may be reduced.

The means for controlling the flow of venting air are arranged to introduce the venting air in a top portion of the venting zone and to evacuate the venting air in a bottom portion of the venting zone, maintaining a flow of venting air essentially from top to bottom.

In one embodiment of the invention, the device comprises an ambient temperature sensor for sensing the ambient temperature outside the device. In this way, the heating in the heating zone may be controlled based on the ambient temperature.

Alternatively or additionally, the device may comprise a package heating temperature sensor for sensing the temperature of the packages entering the heating zone. This may also be used for controlling the heating.

In another embodiment, the device comprises an entry temperature sensor for sensing the temperature of the packages before entry into the sterilization zone. This is another way of enabling control of the heating in the heating zone.

In a preferred embodiment of the invention, the device comprises a feedback circuit for controlling the heating in the heating zone based on the temperature of the packages. In this way, heating to a correct temperature may be ensured and over-heating of the packages may be avoided.

In another embodiment, the device further comprises a condensation detector for detecting condensation in the sterilization zone. Thus, it is possible to mark or discard packages that have been subjected to condensate.

The device of the invention is preferably adapted to sterilize itself internally. Thus, sterile conditions in the device may be ensured in a simple manner.

For sterilizing itself, the device preferably comprises means for heating its interior. This allows efficient sterilization.

The inventive device may comprise a unit for production of the gaseous sterilizing agent, thus making the device self-supporting.

The device may further comprise a filling zone for filling vented packages, and means for maintaining a higher pressure in the filling zone than in the venting zone. This makes it possible to further control the flow of air and gas in the device.

In the method according to the invention for sterilizing packages in production of the packages, said packages having an open end and a closed end, the packages are firstly passed into a heating zone where they are heated to a temperature above the dew point of a sterilizing agent, the gaseous sterilizing agent is used and kept in the gaseous phase throughout the sterilization process and, a venting zone is provided, and a positive pressure is maintained in a sterilization zone in which the sterilization is performed so that it may be ensured that any leakage of gas and air between the sterilization zone and the surrounding heating and venting zones is from the sterilization zone towards the surrounding zones.

Gaseous hydrogen peroxide is preferably used as sterilizing agent. This is a well-known and reliable sterilizing agent.

The heated packages may be passed through an opening in a partitioning separating the heating zone and the sterilization zone into the sterilization zone, where they are subjected to the gaseous sterilizing agent. Since the package has to be passed through a partitioning for entering the sterilization zone, it is easier to control the conditions for sterilization.

Analogously, the sterilized packages may be passed through an opening in a partitioning separating the sterilization zone and the venting zone into the venting zone, where they are subjected to hot sterile air for venting away the sterilizing agent. Thus, the packages may be subjected to distinctly different conditions during sterilization and venting.

The gaseous sterilizing agent in the sterilization zone preferably flows essentially in a direction from the open end of the packages towards the closed end of the packages. Thus, the risk of recontamination of packages may be reduced.

The gaseous sterilizing agent may be introduced in a top portion of the sterilization zone and evacuated in a bottom portion of the sterilization zone, so that a flow of sterilizing agent essentially from top to bottom is maintained. This is a simple way of maintaining a suitable flow for packages standing on their closed end.

In one variant of the method, the venting air in the venting zone flows essentially in a direction from the open end of the packages towards the closed end of the packages. As in the case in the sterilization zone, this reduces the risk of recontamination of packages.

The venting air is preferably introduced in a top portion of the venting zone and evacuated in a bottom portion of the venting zone, so that an air flow essentially from top to bottom is maintained. This is a simple manner of ensuring a suitable flow for packages standing on their closed end.

In a preferred variant of the method, the gaseous sterilizing agent is produced by addition of liquid sterilizing agent to hot air. Thus, the sterilization process may simply and reliably be provided with gaseous sterilizing agent.

An ambient temperature and a concentration of sterilizing agent in the sterilization zone may be measured for controlling the amount of sterilizing agent introduced the sterilization zone.

An ambient temperature may be measured and used for controlling the heating in the heating zone, thus improving control of the sterilization process.

In a variant of the method, a temperature of the packages entering the heating zone is measured and used for controlling the heating in the heating zone. This is an alternative or additional manner of controlling the conditions for the sterilization process.

Further, a temperature of the packages just before they are passed into the sterilization zone may be measured and used for controlling the heating in the heating zone. This is another convenient way of controlling the conditions for the sterilization process.

In one variant of the method of the invention, the temperature and flow of air for production of the gaseous sterilizing agent is controlled based on detection of condensation in the sterilization zone. This makes it easier to avoid condensation.

A higher pressure is preferably maintained in a filling zone for filling vented packages than in the venting zone. Thus, control of flow between the different zones may be improved.

### Brief Description of the Drawings

The invention will be described in more detail with reference to the appended schematic drawings, which show an example of a currently preferred embodiment of the invention.

Fig. 1 is a diagram showing the principles of the sterilizing device of the invention.

Fig. 2 is a perspective view of the sterilizing device.

### Detailed Description of Preferred Embodiments

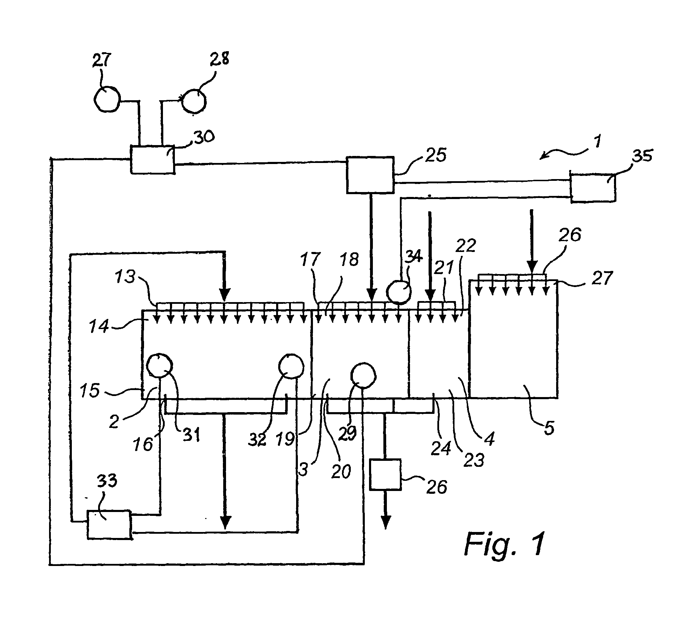

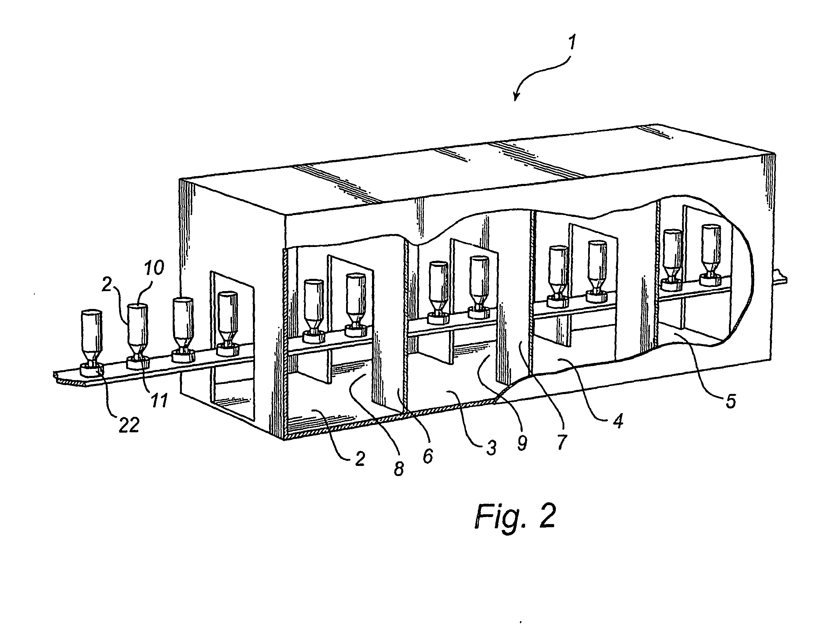

With reference to Fig. 1, the sterilization device 1 has a heating zone 2, a sterilization zone 3, a venting zone 4 and connected thereto a filling zone 5. As may be seen from Fig. 2, the zones 2-5 are separated from each other by partitionings 6-7. In each partitioning 6, 7 there is an opening 6a, 7a. Packages 8 are arranged in holders 9 on a conveyor belt which passes through the zones 2-5. The packages 8 stand on their closed top end 11 with their open bottom end 10 directed upwards.

In the heating zone 2 there are nozzles 13 in a top portion 14 for introduction of hot sterile air. In a bottom portion 15 of the heating zone 2 there are outlets 16 for withdrawing the hot air.

Similarly, there are nozzles 17 for introduction of gaseous hydrogen peroxide in a top portion 18 of the sterilization zone 3. In a bottom portion 19 of the sterilization zone there are outlets 20 for withdrawing hydrogen peroxide.

The venting zone 4 also has nozzles 21 for introducing hot sterile air in a top portion 22. In a bottom portion 23 of the venting zone 4 there are outlets 24 for withdrawing hot air.

The sterilizing device has a gas production unit 25 for producing the gaseous hydrogen peroxide used for sterilization.

In a manner similar to the heating, sterilization and venting zones 2-4, the filling zone 5 has nozzles 26 for introducing sterile air in a top portion 27 of the filling zone.

Included in the device 1 is further a catalyst unit 28 for degrading hydrogen peroxide gas withdrawn from the sterilization zone 3.

The method by which packages are treated in this device 1 will now be described. A package 8 standing on its closed top end 11 in one of the holders 9 is transported by the conveyor belt 10 into the heating zone 2. Here, sterile air with a temperature of approximately 140 °C is introduced in the top portion 14 by means of the nozzles 13. In this manner the package 8 is heated to a temperature above the dew point of the gaseous sterilizing agent to be used in the sterilization zone 3. Thus, it may be ensured that hydrogen peroxide does not condensate on the package 8. The temperature to which the package 8 should be heated depends on the content of hydrogen peroxide in the sterilizing gas, but is normally approximately 70 °C.

Since the heating air is introduced in the top portion 14 of the heating zone 2 and withdrawn through the outlets 16 in the bottom portion 15, an air flow essentially from top to bottom of the heating zone 2 is ensured. Thus, it is also ensured that air flows past the package 8 from the open end 11 towards the closed end 12. This one-way air flow reduces the risk of particles and micro organisms whirling about in the heating zone 2.

Heating in the heating zone 2 is controlled based on a temperature measured on the inside of the package 8 by means of a package heating temperature sensor 31, e.g. an IR temperature sensor. This package heating temperature sensor 31 may also be used in a feed-back circuit providing a safety device for the heating. If, for instance, the conveyor belt 10 transporting the packages is stopped, continued heating in the heating zone 2 might lead to melting of e.g. plastic lids on the packages 8. Therefore, if a predetermined high temperature is measured by the inside temperature sensor, hot air will be by-passed via a shunt, thus not raising the temperature in the heating zone further. When a predetermined low temperature is measured, heating in the heating zone 2 recommences. The predetermined high and low temperature levels are determined based on the properties of the material in the packages 8 and on the hydrogen peroxide content of the sterilizing gas.

The heated package 8 is passed by the conveyor belt 10 through the opening 6a in the partitioning or semi-open wall 6 into the sterilization zone 3. Here, the package 8 is subjected to gaseous hydrogen peroxide introduced at a temperature of approximately 95 °C in the top portion 18 via the nozzles 17. The hydrogen gas is withdrawn through the outlets 20 in the bottom portion 19 and therefore a one-way gas flow is maintained from the top of the sterilization zone 3 towards the bottom. This also means that the flow past the packages 8 is directed from the open end 12 towards the closed end 11. This reduces the risk of recontamination of the packages 8.

Further, an accurate sterilization may be achieved both of the inside and the outside of the package 8. Sterilization of the inside of the package 8 is essential in order to maintain sterility of sterile contents subsequently filled in the package 8. Sterility of the outside of the package improves manageability of the package 8, since the package 8 may be displaced vertically for moving towards and away from an injection nozzle during filling of the package 8 without the risk of particles or micro organisms on the outside of the package 8 contaminating the inside.

Gaseous hydrogen peroxide is introduced and withdrawn at such rate that a positive pressure is maintained in the sterilization zone 3. Thus, it may be ensured that any leakage of gas and air between the sterilization zone 3 and the surrounding heating and venting zones 2, 4 is from the sterilization zone 3 towards the surrounding zones 2, 4. This improves the conditions for an accurate sterilization. Approximately 20 % of the gas leaving the sterilization zone 3 will seep into the heating and venting zones 2, 4, the remaining 80 % being withdrawn through the outlets 20.

The sterilizing gas used in the sterilization zone 3 is produced in the gas production unit 25 by addition of liquid hydrogen peroxide to hot sterile air. The desired hydrogen peroxide concentration in the sterilizing agent may be adjusted by adjustment of the proportion of liquid hydrogen peroxide added to the air. In the example shown, a sterilizing gas containing approximately 35 % hydrogen peroxide is used for sterilization of packages.

Gas withdrawn through the outlets 20 in the bottom portion 19 of the sterilization zone 3 is passed through the catalyst unit 26 for removing hydrogen peroxide from the gas before being evacuated through the evacuation system of the device 1.

The fact that the package 8 is heated above the dew point of the hydrogen peroxide gas before entry into the sterilization zone 3 ensures that hydrogen peroxide does not condensate on the package 8. Therefore, removal of hydrogen peroxide from the package through venting in the venting zone 4 is facilitated. This allows a reliable sterilization even of packages with difficult geometries, e.g. with crevices and pores, without the risk of high residuals of hydrogen peroxide on the package 8 to be filled with an edible content.

The sterilized package 8 is passed through the opening 7a in the partitioning or semi-open wall 7 into the venting zone 4. In the venting zone 4, sterile air is introduced via the nozzles 21 in the top portion 22 at a temperature of approximately 70 °C. The venting air vents away hydrogen peroxide remaining in and on the package 8. Venting is facilitated by the fact that the heating of the package 8 has eliminated condensation of hydrogen peroxide on the package.

The package 8, now sterilized and essentially free from residual hydrogen peroxide, is passed into the filling zone 5 where it is filled with a sterile content, e.g. milk, juice or tomato paste. In order to ensure an air flow essentially from top to bottom, thus minimizing the risk of recontamination of the package 8, sterile air is introduced via the nozzles 26 in the top portion 27 of the filling zone 5. A positive pressure is maintained in the filling zone 5, such that air flow is from the filling 5 outwards and not the other way round.

The filled package 8 is then transported on for sealing and folding of the bottom.

The partitionings between the different zones 2-5 make it possible to control the conditions in each separate zone 2-5 and to control the flow between zones 2-5.

When a production run is finished or when a new run is to begin, the device itself needs to be sterilized. Hot sterile air is then introduced via the nozzles 13 in the heating zone 2 and the inside of the device 1 is heated to approximately 35 °C. When the interior has reached the appropriate temperature, gaseous hydrogen peroxide is introduced in the entire device via the nozzles 17 in the sterilization zone 3. The flow pattern will be different during machine sterilization, i.e. sterilization of the interior of the device 1, as compared to during package sterilization, since the entire interior is to be heated and sterilized. As with package sterilization, sterilizing gas is withdrawn via the catalyst unit 26 for destruction of the hydrogen peroxide.

The sterilization process in the device of the invention is controlled by means of three control loops. In the first control loop, an ambient temperature sensor 27 measures the temperature outside the device 1 and a relative humidity sensor 28 measures the relative humidity outside the device. A concentration meter 29 measures the hydrogen peroxide concentration in the sterilization zone 3. A first control unit 30 receives signals from the ambient temperature sensor 27, the relative humidity sensor 28 and the concentration meter 29 and regulates the amount of hydrogen peroxide added in production of the sterilizing gas. During machine sterilization, the data from the temperature sensor 27, relative humidity sensor 28 and the concentration meter 29 are used for regulating the amount of hydrogen peroxide added to the air stream in the gas production unit 25. In this manner, optimal conditions may be achieved for machine sterilization. During package sterilization, when the hydrogen peroxide concentration of the sterilizing gas is normally significantly higher than during machine sterilization, the relative humidity outside the device 1 is not as important, and therefore only the ambient temperature sensor 27 and the concentration meter 29 are used for regulating the hydrogen peroxide amount added in the gas production unit 25. In this manner, the killing efficiency of the gas may be controlled for purposes of package sterilization.

In the second control loop, a package start temperature sensor 31 is used for measuring the temperature of the packages 8 before entering the device and a package heating temperature sensor 32 is used for measuring the temperature of the packages 8 just before they leave the heating zone 2. Signals from these two temperature sensors 31, 32 are sent to a second control unit 33 which regulates the temperature of the hot air introduced in the heating zone 2 for heating the packages 8 to the required temperature above the dew point of the sterilizing gas. Thus, a correct temperature of the packages 8 may be ensured before they enter the sterilization zone 3.

In the third control loop, a condensation detector 34 detects possible occurrence of condensation in the sterilization zone 3. A signal from the condensation detector 34 is sent to a third control unit 35. If condensation is detected, a signal is sent from the third control unit 35 and used for controlling the temperature and/or flow of hot air in the gas production unit 25. Thus, the temperature of the sterilizing gas and/or the hydrogen peroxide content may be adjusted, such that condensation is avoided. Further, if condensation has been detected, it is possible to mark or discard the packages concerned.

The skilled person will realise that a number of modifications of the invention described above are possible without departing from the scope of the invention as defined in the appended claims.

For instance, only the first of the three control loops described above could be used, or the first control loop could be used in combination with one of the two other control loops.

This first control loop may also be supplemented with a flow meter (not shown) measuring the air flow to the gas production unit connected to the first control unit 30. If the flow of air into the gas production unit 25, and thereby into the sterilization chamber 3, is reduced, e.g. if a filter in the air duct is clogged, the hydrogen peroxide concentration in the sterilizing gas and thus in the sterilization chamber 3 will rise. The first control loop would in such case regulate the amount of hydrogen peroxide added in gas production downwards. However, this may lead to too small a gas flow, so that not all parts of the packages are reached by the sterilizing gas. Therefore, the first control unit 30 may be arranged to sound an alarm at a predetermined low-level flow, so that the low flow may be corrected instead of just lowering the amount of hydrogen peroxide added in the sterilizing gas. Similarly, an alarm signal may be sent at a predetermined high-level flow.

Other means of controlling the heating in the heating zone 2 may also be used. The ambient temperature sensor 27 may be used for determining the temperature outside the device 1. With knowledge of the properties of the materials in the package 8, the required temperature and flow of hot air introduced through the nozzles 13 may be calculated. This may also be combined with the package heating temperature sensor 32 described above for better control and for providing a safety feature.

The package heating temperature sensor 32 may be arranged in the holders 9 holding the packages for measuring the temperature of the packages 8 just before they enter the sterilization zone 3. This temperature sensor 32 may be used for controlling the temperature and flow of the heating air as well as for controlling the safety shunt. It may also be combined with one or both of the previously described temperature sensors for allowing even better control of the heating.

Other sterilizing agents than hydrogen peroxide may also be used, as long as they are suitable for sterilization in the gaseous phase.

## Claims

1. A device for sterilization in production of packages (8), which is adapted for sterilization with a gaseous sterilizing agent kept in the gaseous phase throughout the sterilization process, said device comprising a first heating zone (2), a thereto connected second sterilization zone (3), and a third venting zone (4) connected to said second zone, **characterised in that** it further comprises means for maintaining a higher pressure in the sterilization zone (3) than in the heating zone (2) and venting zone (4).

2. A device as claimed in claim 1, wherein said zones (2, 3, 4) are separated from each other by means of partitionings (6, 7) having openings (6a, 7a) for the passage of packages (8)

3. A device as claimed in claim 1 or 2, which is adapted for sterilization with a gaseous sterilizing agent in the form of gaseous hydrogen peroxide.

4. A device as claimed in any one of the preceding claims, which is adapted to sterilize packages (8) before filling of the packages (8), said packages (8) having an open end (11) and a closed end (12).

5. A device as claimed in claim 4, wherein the heating zone (2) comprises means (13) for heating the packages (8) to a temperature above a dew point of the sterilizing agent used in the sterilization zone (3).

6. A device as claimed in claim 4 or 5, wherein the venting zone (4) comprises means (21, 24) for venting away the sterilizing agent used in the sterilization zone (3) from the packages (8) after sterilization.

7. A device as claimed in any one of claims 4-6, further comprising means (17, 20) for controlling a flow of gaseous sterilizing agent in the sterilization zone (3), such that the gaseous sterilizing agent flows essentially in a direction from the open end (11) of the packages (8) towards the closed end (12) of the packages (8).

8. A device as claimed in claim 7, wherein the means (17, 20) for controlling the flow of gaseous sterilizing agent are arranged to introduce the gaseous sterilizing agent in a top portion (18) of the sterilization zone (3) and to evacuate the gaseous sterilizing agent in a bottom portion (19) of the sterilization zone (3), maintaining a flow of gaseous sterilizing agent essentially from top to bottom.

9. A device as claimed in any one of claims 4-8 further comprising means (21, 24) for controlling a venting air flow in the venting zone (4), such that the venting air flows essentially in a direction from the open end (11) of the packages (8) towards the closed end (12) of the packages (8).

10. A device as claimed in claim 9, wherein the means (21, 24) for controlling the flow of venting air are arranged to introduce the venting air in a top portion (22) of the venting zone (4) and to evacuate the venting air in a bottom portion (23) of the venting zone (4), maintaining a flow of venting air essentially from top to bottom.

11. A device as claimed in any one of claims 4-10, further comprising an ambient temperature sensor for sensing the ambient temperature outside the device (1).

12. A device as claimed in any one of claims 4-11, further comprising an package heating temperature sensor for sensing the temperature of the packages (8) entering the heating zone (2).

13. A device as claimed in any one of claims 4-12, further comprising an entry temperature sensor for sensing the temperature of the packages (8) before entry into the sterilization zone (3).

14. A device as claimed in any one of claims 4-13, further comprising a feedback circuit for controlling the heating in the heating zone (2) based on the temperature of the packages (8).

15. A device as claimed in any one of the preceding claims, further comprising a condensation detector (34) for detecting condensation in the sterilization zone (3)

16. A device as claimed in any one of claims 1-3, which is adapted to sterilize itself (1) internally.

17. A device as claimed in claim 16, further comprising means (13) for heating the interior of the device (1).

18. A device as claimed in any one of the preceding claims, comprising a unit (25) for production of the gaseous sterilizing agent.

19. A device as claimed in any one of the preceding claims, further comprising a filling zone (5) for filling packages (8), and means for maintaining a higher pressure in the filling zone (5) than in the venting zone (4).

20. A method of sterilizing packages (8) in production of the packages (8), said packages (8) having an open end (11) and a closed end (12), wherein the packages are first passed into a heating zone where they are heated to a temperature above the dew point of a sterilizing agent, wherein the gaseous sterilizing agent is used and kept in the gaseous phase throughout the sterilization process and,
wherein a venting zone is provided,
**characterised in that** a positive pressure is maintained in a sterilization zone (3) in which the sterilization is performed so that it may be ensured that any leakage of gas and air between the sterilization zone and the surrounding heating and venting zones is from the sterilization zone towards the surrounding zones.

21. A method as claimed in claim 20, wherein gaseous hydrogen peroxide is used as sterilizing agent.

22. A method as claimed in claim 20, wherein the heated packages (8) are passed through an opening (6a) in a partitioning (6) separating the heating zone (2) and the sterilization zone (3) into the sterilization zone (3), where they are subjected to the gaseous sterilizing agent.

23. A method as claimed in claim 22, wherein the sterilized packages (8) are passed through an opening (7a) in a partitioning (7) separating the sterilization zone (3) and the venting zone (4) into the venting zone (4), where they are subjected to hot sterile air for venting away the sterilizing agent.

24. A method as claimed in claim 22 or 23, wherein the gaseous sterilizing agent in the sterilization zone (3) flows essentially in a direction from the open end (11) of the packages (8) towards the closed end (12) of the packages (8).

25. A method as claimed in claim 24, wherein the gaseous sterilizing agent is introduced in a top portion (18) of the sterilization zone (3) and evacuated in a bottom portion (19) of the sterilization zone (8), so that a flow of sterilizing agent essentially from top to bottom is maintained.

26. A method as claimed in any one of claims 23-25, wherein the venting air in the venting zone (4) flows essentially in a direction from the open end (11) of the packages (8) towards the closed end (12) of the packages (8).

27. A method as claimed in claim 26, wherein the venting air is introduced in a top portion (22) of the venting zone (4) and evacuated in a bottom portion (23) of the venting zone (4), so that an air flow essentially from top to bottom is maintained.

28. A method as claimed in any one of claims 20-27, wherein the gaseous sterilizing agent is produced by addition of liquid sterilizing agent to hot air.

29. A method as claimed in any one of claims 20-28, wherein an ambient temperature and a concentration of sterilizing agent in the sterilization zone (3) are measured and used for controlling the amount of sterilizing agent introduced in the sterilization zone (3).

30. A method as claimed in any one of claims 20-29, wherein an ambient temperature is measured and used for controlling the heating in the heating zone (2).

31. A method as claimed in any one of claims 20-30, wherein a temperature of the packages (8) entering the heating zone (2) is measured and used for controlling the heating in the heating zone (2).

32. A method as claimed in any one of claims 20-31, wherein a temperature of the packages (8) just before they are passed into the sterilization zone (3) is measured and used for controlling the heating in the heating zone (2).

33. A method as claimed in any one of claims 28-31, wherein the temperature and flow of air for production of the gaseous sterilizing agent is controlled based on detection of condensation in the sterilization zone (3).

34. A method as claimed in any one of claims 23-33, wherein a higher pressure is maintained in a filling zone for filling vented packages than in the venting zone (4).

## Patentansprüche

1. Vorrichtung zur Sterilisierung bei der Fertigung von Verpackungen (8), geeignet zur Sterilisierung mit einem gasförmigen Sterilisierungsmittel, das über das Sterilisierungsverfahren hinweg im gasförmigen Stadium gehalten wird, wobei die Vorrichtung eine erste Heizzone (2), eine daran angrenzende zweite Sterilisierungszone (3) und eine dritte, an die zweite Zone angrenzende Lüftungszone (4) umfasst, **dadurch gekennzeichnet, dass** sie ferner Mittel zum Aufrechterhalten eines höheren Drucks in der Sterilisierungszone (3) als in der Heizzone (2) und der Lüftungszone (4) umfasst.

2. Vorrichtung nach Anspruch 1, wobei die Zonen (2, 3, 4) durch Abtrennungen (6, 7) voneinander getrennt sind, die Öffnungen (6a, 7a) für den Durchtritt der Verpackungen (8) aufweisen.

3. Vorrichtung nach Anspruch 1 oder 2, die zur Sterilisierung mit einem gasförmigen Sterilisierungsmittel in Form eines gasförmigen Wasserstoffperoxids geeignet ist.

4. Vorrichtung nach einem der vorhergehenden Ansprüche, die dazu geeignet ist, Verpackungen (8) zu sterilisieren, bevor die Verpackungen (8) befüllt werden, wobei die Verpackungen (8) ein offenes Ende (11) und ein geschlossenes Ende (12) haben.

5. Vorrichtung nach Anspruch 4, wobei die Heizzone (2) Mittel zum Erhitzen der Verpackungen (8) auf eine Temperatur oberhalb des Taupunktes des in der Sterilisierungszone (3) verwendeten Sterilisierungsmittels umfasst.

6. Vorrichtung nach Anspruch 4 oder 5, wobei die Lüftungszone (4) Mittel (21, 24) umfasst, um das Sterilisierungsmittel in der Sterilisierungszone (3) von den Verpackungen (8) nach der Sterilisierung weg zu ventilieren.

7. Vorrichtung nach einem der Ansprüche 4 - 6, ferner umfassend Mittel (17, 20) zum Steuern eines Stroms aus gasförmigem Sterilisierungsmittel in der Sterilisierungszone (3), so dass das gasförmige Sterilisierungsmittel im Wesentlichen in eine Richtung vom offenen Ende (11) der Verpackungen (8) hin zum geschlossenen Ende (12) der Verpackungen (8) strömt.

8. Vorrichtung nach Anspruch 7, wobei die Mittel (17, 20) zum Steuern des Stroms aus gasförmigem Sterilisierungsmittel dazu angeordnet sind, um das gasförmige Sterilisierungsmittel in einen oberen Abschnitt (18) der Sterilisierungszone (3) einzuführen und um das gasförmige Sterilisierungsmittel in einen unteren Abschnitt (19) der Sterilisierungszone (3) zu entleeren, wobei ein Strom aus gasförmigem Sterilisierungsmittel im Wesentlichen von oben nach unten beibehalten wird.

9. Vorrichtung nach einem der Ansprüche 4 - 8, ferner umfassend Mittel (21, 24) zum Steuern eines Lüftungsluftstroms in der Lüftungszone (4), so dass die Lüftungsluft im Wesentlichen in eine Richtung vom offenen Ende (11) der Verpackungen (8) hin zum geschlossenen Ende (12) der Verpackungen (8) strömt.

10. Vorrichtung nach Anspruch 9, wobei die Mittel (21, 24) zum Steuern des Stroms an Lüftungsluft eingerichtet sind, um die Lüftungsluft in einen oberen Abschnitt (22) der Lüftungszone (4) einzuführen und die Lüftungsluft in einen unteren Abschnitt (23) der Lüftungszone (4) zu entleeren, wobei ein Strom aus Lüftungsluft im Wesentlichen von oben nach unten beibehalten wird.

11. Vorrichtung nach einem der Ansprüche 4 - 10, ferner umfassend einen Umgebungstemperatursensor zum Fühlen der Umgebungstemperatur außerhalb der Vorrichtung (1).

12. Vorrichtung nach einem der Ansprüche 4 - 11, ferner umfassend einen Verpackungsheiztemperatursensor, um die Temperatur der Verpackungen (8), die in die Heizzone (2) eintreten, zu fühlen.

13. Vorrichtung nach einem der Ansprüche 4 - 12, ferner umfassend ein Eintrittstemperatursensor zum Fühlen der Temperatur der Verpackungen (8) vor dem Eintritt in die Sterilisierungszone (3).

14. Vorrichtung nach einem der Ansprüche 4 - 13, ferner umfassend eine Rückkopplungsschaltung zum Steuern des Heizens in der Heizzone (2) basierend auf der Temperatur der Verpackungen (8).

15. Vorrichtung nach einem der vorhergehenden Ansprüche, ferner umfassend einen Kondensatdetektor (34) zum Erkennen von Kondensation in der Sterilisierungszone (3).

16. Vorrichtung nach einem der Ansprüche 1 - 3, die dazu ausgelegt ist, sich selbst (1) innen zu sterilisieren.

17. Vorrichtung nach Anspruch 16, ferner umfassend Mittel (13) zum Heizen des Inneren der Vorrichtung (1).

18. Vorrichtung nach einem der vorhergehenden Ansprüche, umfassend eine Einheit (25) zur Herstellung des gasförmigen Sterilisierungsmittels.

19. Vorrichtung nach einem der vorhergehenden Ansprüche, ferner umfassend eine Füllzone (5) zum Befüllen von Verpackungen (8), und Mittel zum Aufrechterhalten eines höheren Drucks in der Füllzone (5) als in der Lüftungszone (4).

20. Verfahren zum Sterilisieren von Verpackungen (8) bei der Herstellung der Verpackungen (8), wobei die Verpackungen (8) ein offenes Ende (11) und ein geschlossenes Ende (12) haben, wobei die Verpackungen zuerst in eine Heizzone befördert werden, wo sie auf eine Temperatur oberhalb des Taupunktes eines Sterilisierungsmittels erhitzt werden, wobei das gasförmige Sterilisierungsmittel über das Sterilisierungsverfahren hinweg im gasförmigen Stadium benutzt und gehalten wird, und
wobei eine Lüftungszone vorgesehen ist,
**dadurch gekennzeichnet, dass** ein Überdruck in einer Sterilisierungszone (3), in der die Sterilisierung erfolgt, aufrechterhalten wird, so dass sichergestellt werden kann, dass jedweder Austritt von Gas und Luft zwischen der Sterilisierungszone und den umgebenden Heiz- und Lüftungszonen von der Sterilisierungszone hin zu den umgebenden Zonen stattfindet.

21. Verfahren nach Anspruch 20, bei dem gasförmiges Wasserstoffperoxid als Sterilisierungsmittel verwendet wird.

22. Verfahren nach Anspruch 20, bei dem die erhitzten Verpackungen (8) durch eine Öffnung (6a) in einer Abtrennung (6) geführt werden, die die Heizzone (2) und die Sterilisierungszone (3) in die Sterilisierungszone (3) trennt, wo sie einem gasförmigen Sterilisierungsmittel ausgesetzt werden.

23. Verfahren nach Anspruch 22, bei dem die sterilisierten Verpackungen (8) durch eine Öffnung (7a) in einer Abtrennung (7), die die Sterilisierungszone (3) und die Lüftungszone (4) trennt, in die Lüftungszone (4) geführt werden, wo sie heißer steriler Luft ausgesetzt werden, um das Sterilisierungsmittel weg zu ventilieren.

24. Verfahren nach Anspruch 22 oder 23, bei dem das gasförmige Sterilisierungsmittel in der Sterilisierungszone (3) im Wesentlichen in eine Richtung vom offenen Ende (11) der Verpackungen (8) hin zum geschlossenen Ende (12) der Verpackungen strömt.

25. Verfahren nach Anspruch 24, wobei das gasförmige Sterilisierungsmittel in einen oberen Abschnitt (18) der Sterilisierungszone (3) eingeführt wird und in einen unteren Abschnitt (19) der Sterilisierungszone (8) entleert wird, so dass ein Strom an Sterilisierungsmittel im Wesentlichen von oben nach unten beibehalten wird.

26. Verfahren nach einem der Ansprüche 23 - 25, bei dem die Lüftungsluft in der Lüftungszone (4) im Wesentlichen in eine Richtung vom offenen Ende (11) der Verpackungen (8) hin zum geschlossenen Ende (12) der Verpackungen (8) strömt.

27. Verfahren nach Anspruch 26, bei dem die Lüftungsluft in einen oberen Abschnitt (22) der Lüftungszone (4) eingeführt wird und in einen unteren Abschnitt (23) der Lüftungszone (4) entleert wird, so dass ein Luftstrom im Wesentlichen von oben nach unten beibehalten wird.

28. Verfahren nach einem der Ansprüche 20 - 27, bei dem das gasförmige Sterilisierungsmittel durch die Zugabe von flüssigem Sterilisierungsmittel zu Heißluft hergestellt wird.

29. Verfahren nach einem der Ansprüche 20 - 28, bei dem eine Umgebungstemperatur und eine Sterilisierungsmittelkonzentration in der Sterilisierungszone (3) gemessen und verwendet werden, um die Menge des in die Sterilisierungszone (3) eingeführten Sterilisierungsmittels zu steuern.

30. Verfahren nach einem der Ansprüche 20 - 29, bei dem eine Umgebungstemperatur gemessen und dazu verwendet wird, um das Heizen in der Heizzone (2) zu steuern.

31. Verfahren nach einem der Ansprüche 20 - 30, bei dem eine Temperatur der Verpackungen (8), die in die Heizzone (2) eintreten, gemessen und dazu verwendet wird, um das Heizen in der Heizzone (2) zu steuern.

32. Verfahren nach einem der Ansprüche 20 - 31, bei dem eine Temperatur der Verpackungen (8), unmittelbar bevor sie in die Sterilisierungszone (3) befördert werden, gemessen und dazu verwendet wird, das Heizen in der Heizzone (2) zu steuern.

33. Verfahren nach einem der Ansprüche 28 - 31, bei dem die Temperatur und der Luftstrom zur Herstellung von gasförmigem Sterilisierungsmittel gesteuert werden, basierend auf dem Erkennen von Kondensation in der Sterilisierungszone (3).

34. Verfahren nach einem der Ansprüche 23 - 33, bei dem ein höherer Druck in der Füllzone zum Befüllen von ventilierten Verpackungen als in der Lüftungszone (4) beibehalten wird.

## Revendications

1. Dispositif pour stérilisation en production d'emballages (8), qui est prévu pour stérilisation avec un agent de stérilisation gazeux maintenu dans la phase gazeuse pendant tout le processus de stérilisation, ce dispositif comprenant une première zone de chauffage (2), une deuxième zone de stérilisation (3) reliée à la précédente, et une troisième zone de ventilation (4) reliée à la dite deuxième zone, **caractérisé en ce qu'**il comprend en outre un moyen de maintien d'une pression plus élevée dans la zone de stérilisation (3) que dans la zone de chauffage (2) et la zone de ventilation (4).

2. Dispositif selon la revendication 1, dans lequel les dites zones (2, 3, 4) sont séparées les unes des autres par des cloisons (6, 7) ayant des ouvertures (6a, 7a) pour le passage des emballages (8).

3. Dispositif selon la revendication 1 ou 2, qui est prévu pour stérilisation avec un agent stérilisant gazeux sous la forme de peroxyde d'hydrogène gazeux.

4. Dispositif selon une quelconque des revendications précédentes, qui est prévu pour stériliser des emballages (8) avant remplissage des emballages (8), les dits emballages (8) ayant une extrémité ouverte (11) et une extrémité fermée (12).

5. Dispositif selon la revendication 4, dans lequel la zone de chauffage (2) comprend des moyens (13) pour chauffer les emballages (8) à une température supérieure à un point de rosée de l'agent stérilisant utilisé dans la zone de stérilisation (3).

6. Dispositif selon la revendication 4 ou 5, dans lequel la zone de ventilation (4) comprend des moyens (21, 24) pour évacuer l'agent stérilisant, utilisé dans la zone de stérilisation (3), hors des emballages (8) après stérilisation.

7. Dispositif selon une quelconque des revendications 4 à 6, comprenant en outre des moyens (17, 20) pour régler un écoulement d'agent stérilisant gazeux dans la zone de stérilisation (3) de sorte que l'agent stérilisant gazeux circule essentiellement dans une direction allant de l'extrémité ouverte (11) des emballages (8) vers l'extrémité fermée (12) des emballages (8).

8. Dispositif selon la revendication 7, dans lequel les moyens (17, 20) pour régler l'écoulement de l'agent stérilisant gazeux sont prévus pour introduire l'agent stérilisant gazeux dans une partie supérieure (18) de la zone de stérilisation (3) et pour évacuer l'agent stérilisant gazeux à une partie inférieure (19) de la zone de stérilisation (3), en maintenant un écoulement d'agent de stérilisation gazeux sensiblement de haut en bas.

9. Dispositif selon une quelconque des revendications 4 à 8, comprenant en outre des moyens (21, 24) pour régler un écoulement d'air de ventilation essentiellement dans la zone de ventilation (4), de sorte que l'air de ventilation s'écoule essentiellement dans une direction allant de l'extrémité ouverte (11) des emballages (8) vers l'extrémité fermée (12) des emballages (8).

10. Dispositif selon la revendication 9, dans lequel Les moyens (21, 24) pour régler l'écoulement d'air de ventilation sont agencés de manière à introduire l'air de ventilation dans une partie supérieure (22) de la zone de ventilation (4) et à évacuer l'air de ventilation à une partie inférieure (23) de la zone de ventilation (4), afin de maintenir un écoulement d'air de ventilation essentiellement du haut en bas.

11. Dispositif selon une quelconque des revendications 4 à 10, comprenant en outre un capteur de température ambiante pour détecter la température ambiante à l'extérieur du dispositif (1).

12. Dispositif selon une quelconque des revendications 4 à 11, comprenant en outre un capteur de température de chauffage des emballages pour détecter la température des emballages (8) qui entrent dans la zone de chauffage (2).

13. Dispositif selon une quelconque des revendications 4 à 12, comprenant en outre un capteur de température d'entrée pour détecter la température des emballages (8) avant l'entrée dans la zone de stérilisation (3).

14. Dispositif selon une quelconque des revendications 4 à 13, comprenant en outre un circuit de réaction pour régler le chauffage dans la zone de chauffage (2) sur la base de la température des emballages (8).

15. Dispositif selon une quelconque des revendications précédentes, comprenant en outre un détecteur de condensation (34) pour détecter une condensation dans la zone de stérilisation (3).

16. Dispositif selon une quelconque des revendications 1 à 3, qui est prévu pour sa propre stérilisation (1) intérieurement.

17. Dispositif selon la revendication 16, comprenant en outre des moyens (13) pour le chauffage de l'intérieur du dispositif (1).

18. Dispositif selon une quelconque des revendications précédentes, comprenant une unité (25) pour la production de l'agent stérilisant gazeux.

19. Dispositif selon une quelconque des revendications précédentes, comprenant en outre une zone de remplissage (5) pour remplir les emballages (8), et des moyens de maintien d'une pression plus élevée dans la zone de remplissage (5) que dans la zone de ventilation (4).

20. Procédé de stérilisation d'emballages (8) dans la production des emballages (8), les dits emballages (8) ayant une extrémité ouverte (11) et une extrémité fermée (12),
dans lequel les emballages passent d'abord dans une zone de chauffage où ils sont chauffés à une température supérieure au point de rosée d'un agent stérilisant,
dans lequel l'agent stérilisant gazeux est utilisé et maintenu dans la phase gazeuse pendant tout le processus de stérilisation, et
dans lequel une zone de ventilation est prévue,
**caractérisé en ce qu'**une pression positive est maintenue dans une zone de stérilisation (3) dans laquelle la stérilisation est effectuée, de sorte qu'on peut assurer qu'une fuite éventuelle de gaz et d'air entre la zone de stérilisation et les zones de chauffage et de ventilation voisines a lieu de la zone de stérilisation vers les zones voisines.

21. Procédé selon la revendication 20, dans lequel le peroxyde d'hydrogène gazeux est utilisé comme agent stérilisant.

22. Procédé selon la revendication 20, dans lequel les emballages chauffés (8) passent à travers une ouverture (6a) ménagée dans une cloison (6) séparant la zone de chauffage (2) et la zone de stérilisation (3), vers la zone de stérilisation (3) où ils sont soumis à l'agent de stérilisation gazeux.

23. Procédé selon la revendication 22, dans lequel les emballages stérilisés (8) passent dans une ouverture (7a) ménagée dans une cloison (7) séparant la zone de stérilisation (3) et la zone de ventilation (4), vers la zone de ventilation (4), où ils sont soumis à de l'air stérile chaud pour chasser l'agent de stérilisation.

24. Procédé selon la revendication 22 ou 23, dans lequel l'agent de stérilisation gazeux dans la zone de stérilisation (3) s'écoule essentiellement dans une direction allant de l'extrémité ouverte (11) des emballages (8) vers l'extrémité fermée (12) des emballages (8).

25. Procédé selon la revendication 24, dans lequel l'agent stérilisant gazeux est introduit dans une partie supérieure (18) de la zone de stérilisation (3) et évacué à une partie inférieure (19) de la zone de stérilisation (3), de sorte qu'un écoulement d'agent stérilisant essentiellement de haut en bas est maintenu.

26. Procédé selon une quelconque des revendications 23 à 25, dans lequel l'air de ventilation dans la zone de ventilation (4) s'écoule essentiellement dans une direction allant de l'extrémité ouverte (11) des emballages (8) vers l'extrémité fermée (12) des emballages (8).

27. Procédé selon la revendication 26, dans lequel l'air de ventilation est introduit dans une partie supérieure (22) de la zone de ventilation (4) et évacué à une partie inférieure (23) de la zone de ventilation (4), de sorte qu'un écoulement d'air essentiellement de haut en bas est maintenu.

28. Procédé selon une quelconque des revendications 20 à 27, dans lequel l'agent de stérilisation gazeux est produit par addition d'un agent de stérilisation liquide à de l'air chaud.

29. Procédé selon une quelconque des revendications 20 à 28, dans lequel une température ambiante et une concentration d'agent de stérilisation dans la zone de stérilisation (3) sont mesurées et utilisées pour régler la quantité d'agent de stérilisation introduite dans la zone de stérilisation (3).

30. Procédé selon une quelconque des revendications 20 à 29, dans lequel une température ambiante est mesurée et utilisée pour le réglage du chauffage dans la zone de chauffage (2).

31. Procédé selon une quelconque des revendications 20 à 30, dans lequel une température des emballages (8) entrant dans la zone de chauffage (2) est mesurée et utilisée pour le réglage du chauffage dans la zone de chauffage (2).

32. Procédé selon une quelconque des revendications 20 à 31, dans lequel une température des emballages (8) juste avant qu'ils entrent dans la zone de stérilisation (3) est mesurée et utilisée pour le réglage du chauffage dans la zone de chauffage (2).

33. Procédé selon une quelconque des revendications 28 à 31, dans lequel la température et le débit d'air pour la production de l'agent de stérilisation gazeux sont contrôlés sur la base de la détection de la condensation dans la zone de stérilisation (3).

34. Procédé selon une quelconque des revendications 23 à 33, dans lequel une pression plus élevée est maintenue dans une zone de remplissage, pour remplir les emballages purgés, que dans la zone de ventilation (4).
